# EUROPEAN PATENT SPECIFICATION

(11) **EP 2 917 232 B1**
(45) Date of publication and mention of the grant of the patent: **01.03.2017**
(21) Application number: 13795002.8
(22) Date of filing: 11.11.2013
(51) Int. Cl.: C07K 14/755, A61K 38/00, A61K 39/00, A61K 38/37

(54) **FACTOR VIII-DERIVED PEPTIDES FOR USE IN THE TREATMENT OF HAEMOPHILIA A**
PEPTIDE VON FAKTOR VIII ZUR VERWENDUNG BEI DER BEHANDLUNG VON HÄMOPHILIA A
PEPTIDES DÉRIVÉS DU FACTEUR VIII POUR LEUR UTILISATION DANS LE TRAITEMENT DE L'HÉMOPHILIE A

(30) Priority: 12.11.2012 GB 201220328; 19.09.2013 GB 201316660
(43) Date of publication of application: 16.09.2015
(73) Proprietor: Apitope International NV, 3590 Diepenbeek (BE)
(72) Inventor: WRAITH, David, Bristol BS8 1TD (GB); STREETER, Heather, Bristol BS8 1TD (GB)
(74) Representative: D Young & Co LLP
(86) International application number: PCT/IB2013/060060
(87) International publication number: WO 2014/072958

(56) References cited:
- WO-A1-2009/071886
- WO-A2-2009/095646
- WO-A2-2010/133834
- FR-A1- 2 842 812

## Description

### FIELD OF THE INVENTION

The present invention relates to peptides, at least part of which is derivable from factor VIII (FVIII). The peptides can be used to reduce or prevent factor VIII inhibitor antibody formation, for example in haemophilia A treatment and acquired haemophilia.

### BACKGROUND TO THE INVENTION

### HAEMOPHILIA

Haemophilia belongs to a group of inheritable blood disorders that includes haemophilia A, haemophilia B (Christmas disease) and Von Willebrand's disease.

In haemophilia, the blood's ability to clot is severely reduced because an essential clotting factor is partly or completely missing, resulting in increased bleeding time.

Haemophilia A is a deficiency of the clotting factor VIII, whereas Haemophilia B is a deficiency of clotting factor IX. In both diseases, the faulty gene is found on the X chromosome, so the conditions are X-linked. Haemophilia A is five times more common than haemophilia B.

Haemophilia is a lifelong inherited genetic condition, which affects females as carriers and males who inherit the condition. About a third of new diagnoses are where there is no previous family history. It appears world-wide and occurs in all racial groups. About 6,000 people are affected with haemophilia in the UK.

Haemophiliacs bleed for a prolonged period following injury. External injuries such as cuts and grazes do not usually pose serious problems: it is often possible to stop bleeding by applying a degree of pressure and covering the affected area (e.g with a plaster).

The main problem is internal bleeding into joints, muscles and soft tissues, which can occur spontaneously. Internal bleeding, such are haemorrhages into the brain, is very difficult to manage and can be fatal. Repeated bleeding in the joints causes acute pain and can cause arthritis and/or long-term joint damage leading to disability.

Treatment for haemophilia is usually by replacement of the missing clotting factor. In mild or moderate haemophilia injections may be given at the time a bleed occurs (on-demand therapy). However, in severe haemophilia regular prophylactic injections are given to help the blood to clot and minimise the likelihood of long term joint damage.

A potentially serious complication of coagulation factor replacement therapy for haemophilia A is the development of antibodies that neutralise the procoagulant function of factor VIII. Factor VIII inhibitors occur in approximately 25% of those with severe haemophilia A. Since patients with congenital haemophilia A can be genetically deficient in FVIII, the synthesis of inhibitors is an alloimmune response to the foreign protein administered to prevent or treat bleeding episodes.

CD4+ T cells play a central role in the immune response to FVIII. After being taken up by antigen-presenting cells (APCs), FVIII undergoes proteolytic degradation into peptide fragments (Reding et al (2006) Haemophilia 12(supp 6) 30-36). These peptides are then presented on the surface of the APC in association with MHC class II molecules. This complex is then recognised by the T cell receptor of a CD4+ cell specific for FVIII. In the presence of the appropriate costimulatory signals, this recognition ultimately causes the CD4+ cell to direct the synthesis of antibodies by B cells.

The incidence of inhibitor formation initially increases with the number of factor VIII treatments, but appears to plateau after 50-100 exposure days. Inhibitor formation is much more common in severe haemophilia than in moderate or mild disease and some molecular defects, most clearly large deletions and nonsense mutations in the factor VIII light chain, appear to predispose to inhibitor formation. Parameters such as the concentration, type (purified or recombinant) of replacement factor, and treatment history may also affect the likelihood of antibody production.

The management of haemophilia patients with inhibitors is an ongoing challenge. Immune tolerance induction (ITI) using a desensitization technique is successful in some patients with alloantibodies against factor VIII. This therapeutic approach requires ongoing exposure to factor replacement therapy, so is a long-term strategy.

Although ITI can be successful, a significant proportion (about 30%) of patients fails to respond to ITI. Patients with high inhibitor titres are much less likely to respond to treatment. Another significant contributing factor is age at the start of commencing ITI, with greatly decreased success rates when the patient is older than 20 (Hay et al (2005) Seminars in Thrombosis and Hemostasis 32:15-21)

When ITI therapy is unsuccessful, the inhibitor generally persists for life, and because such patients are usually high-responders, it is necessary to treat episodes of bleeding with FVIII bypassing products, such as activated prothombin complex concentrates (FEIBA™), and recombinant-activated FVII. However, the use of such agents is associated with adverse events such as disseminated intravascular coagulation, acute myocardial infarction, pulmonary embolus and thromboses (Acharya and DiMichele (2006) Best Practice & Research Clinical Haematology 19:51-66).

Immunosuppressive therapy is sometimes used for patients who fail to response to ITI. Treatment includes administration of immunosuppressive drugs such as cyclophosphamide, prednisone, azathioprine and cyclosporine which non-specifically target the immune system. These treatments can have side-effects associated with general immunosuppression.

There is renewed interest on selective B cell depletion using Rituximab™, a humanised monoclonal antibody to B cell CD20 antigen. However, infusion reactions, serum sickness and opportunistic infections have occurred in some children treated with this drug (DiMichele (2007) J Thromb Haemost 5:143-50).

### ACQUIRED HAEMOPHILIA

Acquired haemophilia is a rare autoimmune condition which affects between 1 and 4 people in every million. In this condition, subjects who are not born with haemophilia develop antibodies against one of the clotting factors such as factor VIII. It is thought that pregnancy and autoimmune diseases such as rheumatoid arthritis and cancer may increase the risk of developing acquired haemophilia. Although there are differences in the underlying immune mechanisms leading to their production, the clinical manifestations of FVIII inhibitors produced in response to coagulation factor replacement therapy and those produced in acquired haemophilia are similar.

Acquired haemophiliac patients have a mortality rate that approaches 25%, partly because of the association of acquired inhibitors with severe bleeding complications. The therapy of acquired autoantibody inhibitors is based primarily on the need to control or prevent acute hemorrhagic complications, which frequently are life and limb threatening and secondarily to eradicate the autoantibody to restore normal coagulation.

Some bleeds associated with low titre autoantibody inhibitors (< 5 Bethesda Units) may be treated effectively with FVIII concentrates administered at high doses. Porcine FVIII concentrate was formerly considered a critical first-line therapy for acquired haemophilia-related bleeding since it was the only replacement therapy that provided an opportunity to actually measure post-infusion FVIII coagulation activity levels in the laboratory. The product was removed from the marketplace in 2004 because of contamination of the porcine plasma pools by porcine parvovirus. Now, "bypassing" agents are most commonly used, but potential risks of thrombogenicity exist and there is only about 80% efficacy for each product. Plasma exchange via plasmapheresis and extracorporeal immunoadsorption may be necessary to temporarily reduce the inhibitor titer enough for bypassing agents or FVIII replacement to provide adequate hemostasis.

Eradication of autoantibody inhibitors depends on immunosuppressive measures, such as: (1) administration of corticosteroids with 30%-50% efficacy in 3-6 weeks; (2) use of cytotoxic and myelosuppressive chemotherapeutic agents, e.g., cyclophosphamide, cyclosporine, 2-chlorodeoxyadenosine; (3) immunomodulation with intravenous immunoglobulin; and (4) selective B-lymphocyte depletion with rituximab. Rituximab™ responders may require concurrent use of steroids and relapses may respond to retreatment.

Thus, all currently available methods for reducing alloantibody production associated with haemophilia A treatment, and autoantibody production in acquired haemophilia, have shortcomings. There is therefore a need for improved methods to address the issue of anti-FVIII antibodies in haemophilia A and acquired haemophilia.

The present inventors have found that it is possible to prevent FVIII inhibitor antibody formation by pre-tolerising the patient with FVIII-derived peptides and to treat conditions such as haemophilia by administration of FVIII-derived peptides in order to reduce FVIII inhibitor antibodies.

### SUMMARY OF ASPECTS OF THE INVENTION

The first aspect of the present invention, therefore, relates to a peptide, at least part of the sequence of which is derivable from FVIII, which is capable of inducing or restoring tolerance to FVIII.

In a first embodiment of the first aspect of the invention, the invention provides a peptide comprising the FVIII-derived sequence DNIMVTFRNQASRPY which has one of the following sequences:
Lys-Lys-Gly-Asp-Asn-Ile-Met-Val-Thr-Phe-Arg-Asn-Gln-Ala-Ser-Arg-Pro-Tyr-Gly-Lys-Lys (SEQ ID No. 17)
Lys-Lys-Gly-Asp-Asn-Ile-Met-Val-Thr-Phe-Arg-Asn-Gln-Ala-Ser-Arg-Pro-Tyr-Gly-Lys-Glu (SEQ ID No. 18)
Lys-Lys-Gly-Asp-Asn-Ile-Met-Val-Thr-Phe-Arg-Asn-Gln-Ala-Ser-Arg-Pro-Tyr-Gly-Glu-Lys (SEQ ID No. 19)
Lys-Glu-Gly-Asp-Asn-Ile-Met-Val-Thr-Phe-Arg-Asn-Gln-Ala-Ser-Arg-Pro-Tyr-Gly-Lys-Lys (SEQ ID No. 25)
Lys- Glu-Gly-Asp-Asn-Ile-Met-Val-Thr-Phe-Arg-Asn-Gln-Ala-Ser-Arg-Pro-Tyr-Gly-Lys-Glu (SEQ ID No. 26)
Lys- Glu-Gly-Asp-Asn-Ile-Met-Val-Thr-Phe-Arg-Asn-Gln-Ala-Ser-Arg-Pro-Tyr-Gly-Glu-Lys (SEQ ID No. 27)
Glu-Lys-Gly-Asp-Asn-Ile-Met-Val-Thr-Phe-Arg-Asn-Gln-Ala-Ser-Arg-Pro-Tyr-Gly-Lys-Lys (SEQ ID No. 29)
Glu-Lys-Gly-Asp-Asn-Ile-Met-Val-Thr-Phe-Arg-Asn-Gln-Ala-Ser-Arg-Pro-Tyr-Gly-Lys-Glu (SEQ ID No. 30) and
Glu-Lys-Gly-Asp-Asn-Ile-Met-Val-Thr-Phe-Arg-Asn-Gln-Ala-Ser-Arg-Pro-Tyr-Gly-Glu-Lys (SEQ ID No. 31).

In a second embodiment, the present invention provides a peptide comprising the FVIII-derived sequence PRCLTRYYSSFVNME which has one of the following sequences:
Lys-Lys-Gly-Pro-Arg-Cys-Leu-Thr-Arg-Tyr-Tyr-Ser-Ser-Phe-Val-Asn-Met-Glu-Gly-Lys-Lys (SEQ ID No. 1)
Lys-Lys-Gly-Pro-Arg-Cys-Leu-Thr-Arg-Tyr-Tyr-Ser-Ser-Phe-Val-Asn-Met-Glu-Gly-Lys-Glu (SEQ ID No. 2)
Lys-Lys-Gly-Pro-Arg-Cys-Leu-Thr-Arg-Tyr-Tyr-Ser-Ser-Phe-Val-Asn-Met-Glu-Gly-Glu-Lys (SEQ ID No. 3)
Glu-Glu-Gly-Pro-Arg-Cys-Leu-Thr-Arg-Tyr-Tyr-Ser-Ser-Phe-Val-Asn-Met-Glu-Gly-Lys-Lys (SEQ ID No. 5)
Glu-Glu-Gly-Pro-Arg-Cys-Leu-Thr-Arg-Tyr-Tyr-Ser-Ser-Phe-Val-Asn-Met-Glu-Gly-Glu-Lys (SEQ ID No. 7)
Lys-Glu-Gly-Pro-Arg-Cys-Leu-Thr-Arg-Tyr-Tyr-Ser-Ser-Phe-Val-Asn-Met-Glu-Gly-Lys-Lys (SEQ ID No. 9)
Lys- Glu-Gly-Pro-Arg-Cys-Leu-Thr-Arg-Tyr-Tyr-Ser-Ser-Phe-Val-Asn-Met-Glu-Gly-Lys-Glu (SEQ ID No. 10)
Lys- Glu-Gly-Pro-Arg-Cys-Leu-Thr-Arg-Tyr-Tyr-Ser-Ser-Phe-Val-Asn-Met-Glu-Gly-Glu-Lys (SEQ ID No. 11) and
Glu-Lys-Gly-Pro-Arg-Cys-Leu-Thr-Arg-Tyr-Tyr-Ser-Ser-Phe-Val-Asn-Met-Glu-Gly-Lys-Lys (SEQ ID No. 13).

In a second aspect, the present invention provides a composition comprising a plurality of peptides, including one or more peptide(s) according to the first aspect the invention.

The composition may comprise at least one peptide according to the first embodiment and at least one peptide according to the second embodiment of the first aspect of the invention.

The composition may comprise a peptide having SEQ ID No. 1 and a peptide having SEQ ID No. 17.

The composition may be in the form of a kit, in which the plurality of peptides are provided separately for separate, subsequent, sequential or simultaneous administration.

The peptide or a composition of the invention may be for use in suppressing, reducing, or preventing the development of factor VIII inhibitor antibodies.

Described herein is also the use of such a peptide or composition in the manufacture of a medicament to suppress, reduce or prevent the development of factor VIII inhibitor antibodies.

Described herein is also a method for suppressing, preventing or reducing the development of Factor VIII inhibitor antibodies in a subject, which comprises the step of administration of such a peptide or composition to the subject.

The subject may be deficient in FVIII. In particular the subject may have haemophilia A, and may be, or be about to, undergo factor VIII replacement therapy.

Alternatively the subject may have, or be at risk from contracting, acquired haemophilia.

Factor VIII inhibitors are found more frequently in individuals expressing HLA-DR2. The subject treated by the method of the invention may therefore be HLA-DR2 positive.

### DESCRIPTION OF THE FIGURES

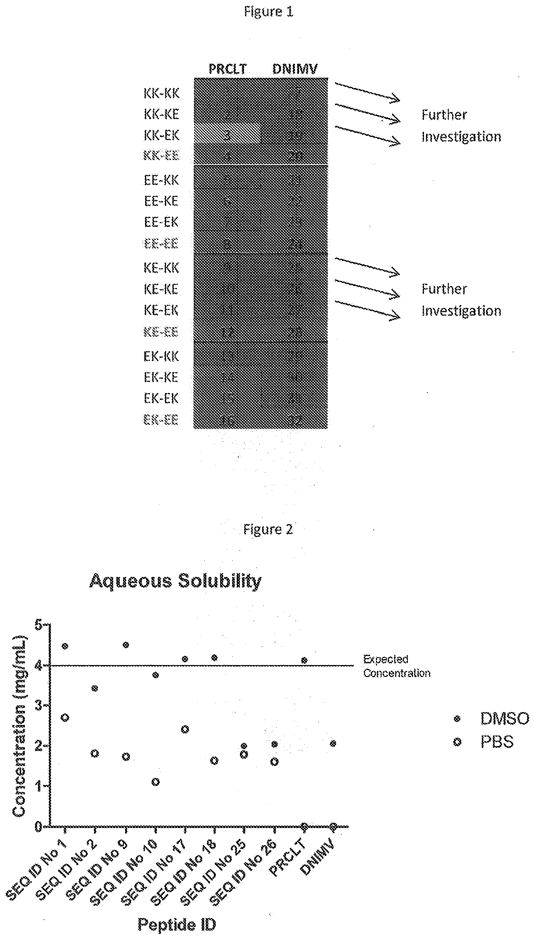

**Figure 1****: Solubility of modified FVIII peptides**
   A total of 32 peptides were tested: 16 of which were based on the FVIII peptide PRCLTRYYSSFVNME ("PRCLT") (SEQ ID No. 1,2,3,4,5,6,7,8,9,10,11,12,13); and 16 of which were based on the FVIII peptide DNIMVTFRNQASRPY ("DNIMV") (SEQ ID No. 17,18,19,20,21,22,23,245,26,27,28,29,30,31,32). Their solubility was tested relative to an unrelated control peptide (4Y) which is known to be sufficiently soluble for application as a tolerising antigen. In the diagram, green represents a peptide which is at least as soluble as 4Y, orange represents a peptide which is not quite as soluble as 4Y but is fairly close, and red represents a peptide which is not as soluble as 4Y. Peptides coded green were as soluble as control peptide 4Y, peptides coded red were poorly soluble, and one peptide coded amber had intermediate solubility.
**Figure 2****: Solubility of tagged peptides in aqueous solution.**
   The concentration of peptide in solution following dissolution in DMSO (control) or PBS was determined spectrophotometrically and compared to the expected value of 4mg/mL. A greater disparity between the actual and expected concentration corresponds to lower relative solubility.
**Figure 3****: The tagged peptides act as apitopes**
   Antigen presentation assays with fresh and fixed antigen presenting cells were conducted to investigate whether the tagged peptides, like the parent peptides PRCLT and DNIMV, are capable of binding to an MHC molecule and being presented to a T cell without antigen processing. (a) PRCLT peptides; (b) DNIMV peptides.

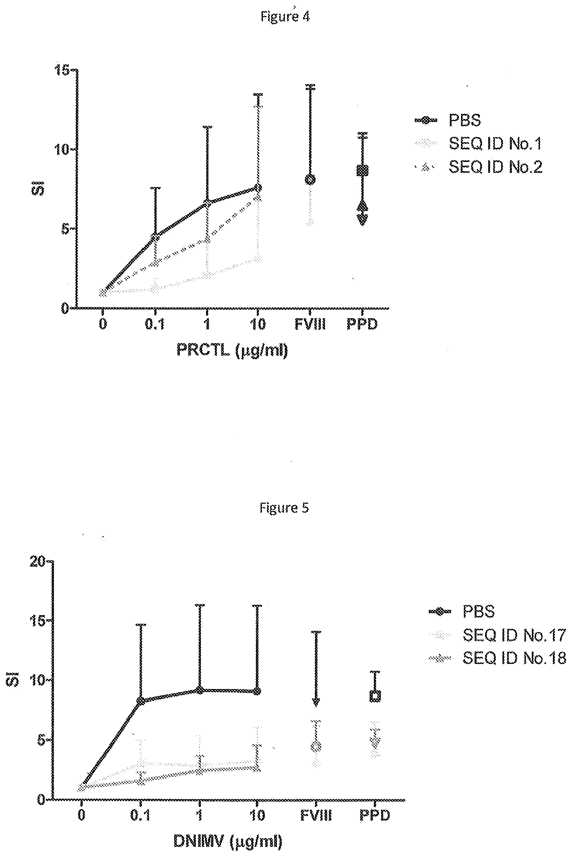

**Figure 4****: Peptides having SEQ ID 1 and SEQ ID 2 regulate a T cell response to PRCLT**
   The figure shows recall responses from HLA-DR2 transgenic mice treated with either SEQ ID 1, SEQ ID 2 or control (PBS) and primed with PRCLT. T cell stimulation indices are shown across a range of peptide concentrations and against FVIII and the control PPD
**Figure 5****: Peptides having SEQ ID 17 and SEQ ID 18 regulate a T cell response to DNIMV.**
   The figure shows recall responses from HLA-DR2 transgenic mice treated with either SEQ ID 17, SEQ ID 18 or control (PBS) and primed with DNIMV. T cell stimulation indices are shown across a range of peptide concentrations and against FVIII and the control PPD.

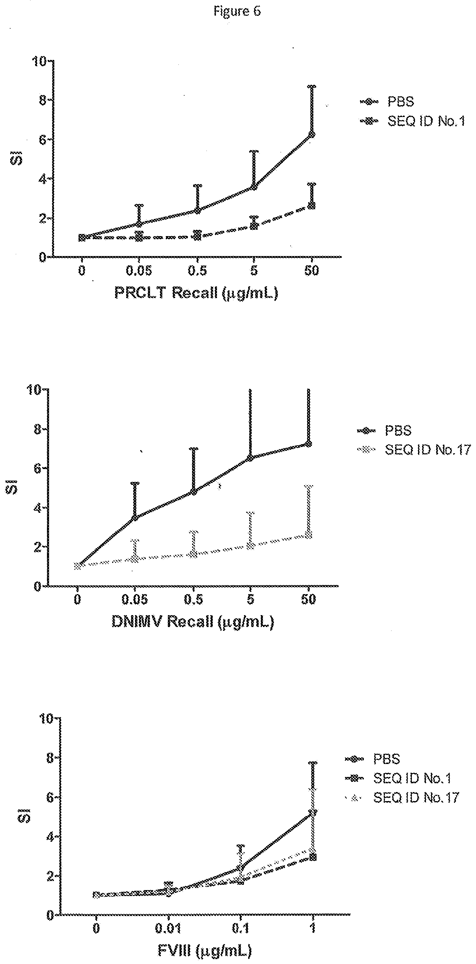

**Figure 6****: Peptides having SEQ ID 1 and SEQ ID 17 regulate a T cell response to their parental peptides.**
   The figure shows recall responses from HLA-DR2 transgenic mice treated with either SEQ ID1 or SEQ ID 17 or control (PBS) and primed with PRCLT and DNIMV and recalled in vitro with PRCLT, DNIMV or FVIII. T cell stimulation indices are shown across a range of peptide concentrations and against FVIII. There is a significant inhibition of T cell responses to the parental peptides.

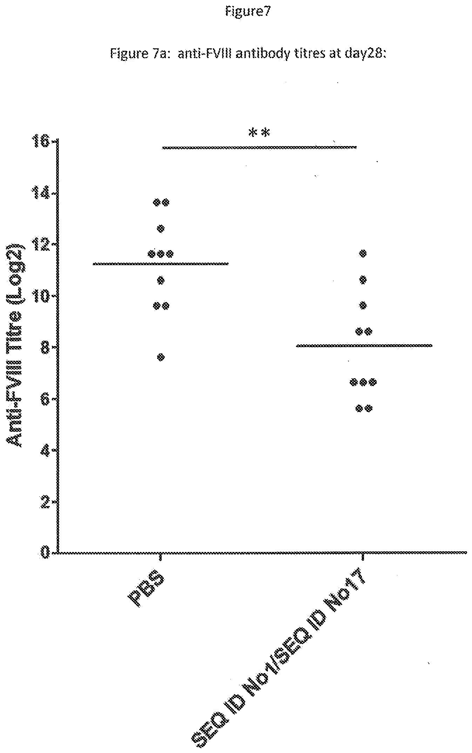

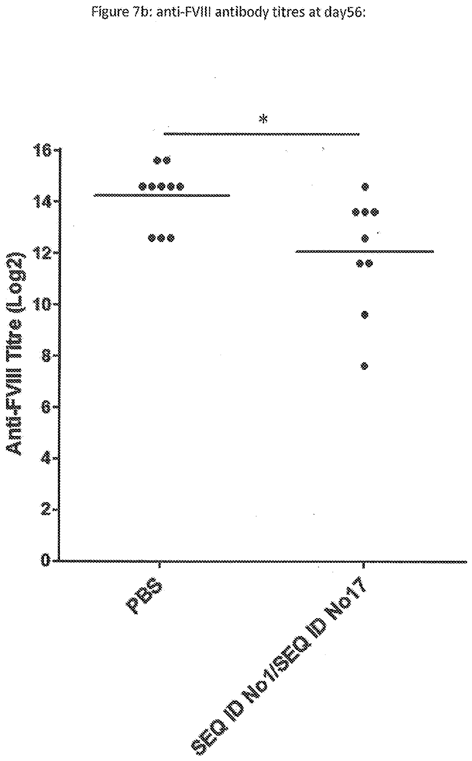

**Figure 7****: A combination of a peptide having SEQ ID No. 1 and a peptide having SEQ ID No. 17 inhibits anti-FVIII antibody production *in vivo.***
   The figure shows end point anti-FVIII antibody titres of mice treated with a combination of SEQ ID 1 and SEQ ID 17 (or PBS control) and immunised with 8 weekly FVIII immunisations
   Figure 7a: results at day28
   Figure 7b: results at day56

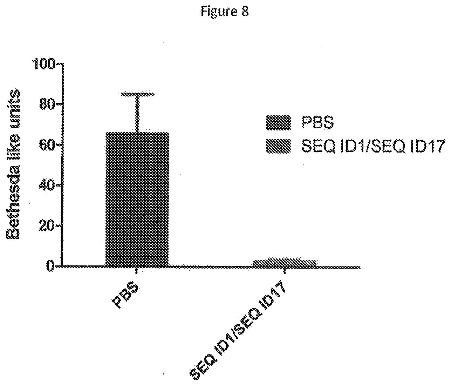

**FIGURE 8****: A combination of a peptide having SEQ ID No. 1 and a peptide having SEQ ID No. 17 prevents neutralising anti-FVIII antibody production in vivo upon FVIII exposure**
   The figure shows the levels of neutralising anti-FVIII antibodies in mice treated with a combination of SEQ ID 1 and SEQ ID 17 (or PBS control) prior to and during 8 weekly FVIII immunisations. At day56 there is a significant reduction of neutralising antibodies in peptide treated mice.

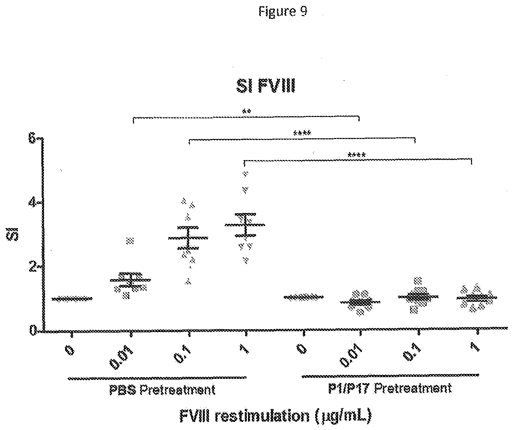

**Figure 9****: A combination of a peptide having SEQ ID No. 1 and a peptide having SEQ ID No. 17 inhibits T cell responses to FVIII.**
   The figure shows recall responses from HLA-DR2 transgenic mice treated with a combination of SEQ ID 1 and, SEQ ID 17 or control (PBS) and primed with DNIMV and PRCLT. T cell stimulation indices are shown across a range of FVIII concentrations.

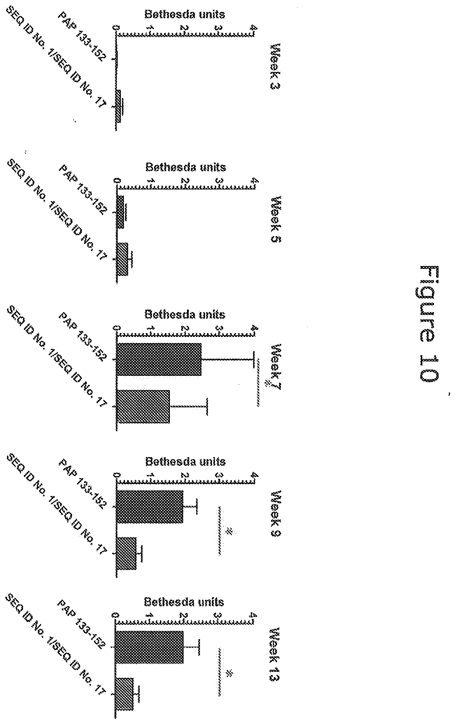

**FIGURE 10****: A combination of a peptide having SEQ ID No. 1 and a peptide having SEQ ID No. 17 suppresses neutralising anti-FVIII antibody generation in a therapeutic animal model with an ongoing FVIII immune response**
   The figure shows the levels of neutralising anti-FVIII antibodies in mice treated with a combination of SEQ ID 1 and SEQ ID 17 or prostatic acid phosphatase (PAP) 133-152 control peptide (sequence as described in PCT/US2006/031961, SEQ ID No. 15) after a FVIII immune response and anti-FVIII antibody production was induced. Mice received peptide treatment three weeks after rFVIII immunisation and prior to a boost immunization with rFVIII. Two weeks after the FVIII boost immunisation (week 7), there is a significant reduction of neutralizing anti-FVIII antibodies in peptide treated mice which is maintained during the course of the experiment for up to week 13.

### DETAILED DESCRIPTION

### PEPTIDE

The present invention relates to a peptide.

The term "peptide" is used in the normal sense to mean a series of residues, typically L-amino acids, connected one to the other typically by peptide bonds between the α-amino and carboxyl groups of adjacent amino acids. The term includes modified peptides and synthetic peptide analogues.

The peptide of the present invention may be made using chemical methods (Peptide Chemistry, A practical Textbook. Mikos Bodansky, Springer-Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage of a peptide from factor VIII followed by modification of one or both ends. The composition of a peptide may be confirmed by amino acid analysis or sequencing (e.g., the Edman degradation procedure).

For practical purposes, there are various other characteristics which the peptide may show. For example, it is important that the peptide is sufficiently stable *in vivo* to be therapeutically useful. The half-life of the peptide *in vivo* may be at least 10 minutes, 30 minutes, 4 hours, or 24 hours.

The peptide may also demonstrate good bioavailability *in vivo.* The peptide may maintain a conformation *in vivo* which enables it to bind to an MHC molecule at the cell surface without due hindrance.

### APITOPES

In an adaptive immune response, T lymphocytes are capable of recognising internal epitopes of a protein antigen. Antigen presenting cells (APC) take up protein antigens and degrade them into short peptide fragments. A peptide may bind to a major histocompatibility complex (MHC) class I or II molecule inside the cell and be carried to the cell surface. When presented at the cell surface in conjunction with an MHC molecule, the peptide may be recognised by a T cell (via the T cell receptor (TCR)), in which case the peptide is a T cell epitope.

An epitope is thus a peptide derivable from an antigen which is capable of binding to the peptide-binding groove of a MHC class I or II molecule and be recognised by a T cell.

The minimal epitope is the shortest fragment derivable from an epitope, which is capable of binding to the peptide-binding groove of a MHC class I or II molecule and being recognised by a T cell. For a given immunogenic region, it is typically possible to generate a "nested set" of overlapping peptides which act as epitopes, all of which contain the minimal epitope but differ in their flanking regions.

By the same token, it is possible to identify the minimal epitope for a particular MHC molecule:T cell combination by measuring the response to truncated peptides. For example if a response is obtained to the peptide comprising residues 1-15 in the overlapping library, sets which are truncated at both ends (i.e. 1-14, 1-13, 1-12 etc. and 2-15, 3-15, 4-15 etc.) can be used to identify the minimal epitope.

The present inventors have previously determined that there is a link between the capacity of a peptide to bind to an MHC class I or II molecule and be presented to a T cell without further antigen processing, and the peptide's capacity to induce tolerance *in vivo* (WO 02/16410). If a peptide is too long to bind the peptide binding groove of an MHC molecule without further processing (e.g. trimming), or binds in an inappropriate conformation then it will not be tolerogenic *in vivo.* If, on the other hand, the peptide is of an appropriate size and conformation to bind directly to the MHC peptide binding groove and be presented to a T cell, then this peptide can be predicted to be useful for tolerance induction.

It is thus possible to investigate the tolerogenic capacity of a peptide by investigating whether it can bind to an MHC class I or II molecule and be presented to a T cell without further antigen processing *in vitro.*

The peptides of the present invention are apitopes (Antigen Processing-Independent epiTOPES) in that they are capable of binding to an MHC class II molecule and stimulating a response from factor VIII specific T cells without further antigen processing. Such apitopes can be predicted to cause tolerance to FVIII, following the rule-based method described in WO 02/16410.

A peptide of the present invention may be any length that is capable of binding to an MHC class I or II molecule without further processing. Typically, the peptide of the present invention is capable of binding MHC class II.

Peptides that bind to MHC class I molecules are typically 7 to 13, more usually 8 to 10 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations is peptide length are accommodated by a kinking in the peptide backbone, often at proline or glycine residues that allow the required flexibility.

Peptides which bind to MHC class II molecules are typically between 8 and 20 amino acids in length, more usually between 10 and 17 amino acids in length, and can be longer (for example up to 40 amino acids). These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove.

### PEPTIDE SEQUENCES

The first embodiment of the invention relates to a peptide comprising a FVIII-derived sequence. FVIII-derived sequences which were investigated in the Examples have the following sequences
SEQ ID No. 1: Lys-Lys-Gly-Pro-Arg-Cys-Leu-Thr-Arg-Tyr-Tyr-Ser-Ser-Phe-Val-Asn-Met-Glu-Gly-Lys-Lys
SEQ ID No. 2: Lys-Lys-Gly-Pro-Arg-Cys-Leu-Thr-Arg-Tyr-Tyr-Ser-Ser-Phe-Val-Asn-Met-Glu-Gly-Lys-Glu
SEQ ID No. 3: Lys-Lys-Gly-Pro-Arg-Cys-Leu-Thr-Arg-Tyr-Tyr-Ser-Ser-Phe-Val-Asn-Met-Glu-Gly-Glu-Lys
SEQ ID No. 4: Lys-Lys-Gly-Pro-Arg-Cys-Leu-Thr-Arg-Tyr-Tyr-Ser-Ser-Phe-Val-Asn-Met-Glu-Gly-Glu-Glu
SEQ ID No. 5: Glu-Glu-Gly-Pro-Arg-Cys-Leu-Thr-Arg-Tyr-Tyr-Ser-Ser-Phe-Val-Asn-Met-Glu-Gly-Lys-Lys
SEQ ID No. 6: Glu-Glu-Gly-Pro-Arg-Cys-Leu-Thr-Arg-Tyr-Tyr-Ser-Ser-Phe-Val-Asn-Met-Glu-Gly-Lys-Glu
SEQ ID No. 7: Glu-Glu-Gly-Pro-Arg-Cys-Leu-Thr-Arg-Tyr-Tyr-Ser-Ser-Phe-Val-Asn-Met-Glu-Gly-Glu-Lys
SEQ ID No. 8: Glu-Glu-Gly-Pro-Arg-Cys-Leu-Thr-Arg-Tyr-Tyr-Ser-Ser-Phe-Val-Asn-Met-Glu-Gly-Glu-Glu
SEQ ID No. 9: Lys-Glu-Gly-Pro-Arg-Cys-Leu-Thr-Arg-Tyr-Tyr-Ser-Ser-Phe-Val-Asn-Met-Glu-Gly-Lys-Lys
SEQ ID No. 10: Lys-Glu-Gly-Pro-Arg-Cys-Leu-Thr-Arg-Tyr-Tyr-Ser-Ser-Phe-Val-Asn-Met-Glu-Gly-Lys-Glu
SEQ ID No. 11: Lys-Glu-Gly-Pro-Arg-Cys-Leu-Thr-Arg-Tyr-Tyr-Ser-Ser-Phe-Val-Asn-Met-Glu-Gly-Glu-Lys
SEQ ID No. 12: Lys-Glu-Gly-Pro-Arg-Cys-Leu-Thr-Arg-Tyr-Tyr-Ser-Ser-Phe-Val-Asn-Met-Glu-Gly-Glu-Glu
SEQ ID No. 13: Glu-Lys-Gly-Pro-Arg-Cys-Leu-Thr-Arg-Tyr-Tyr-Ser-Ser-Phe-Val-Asn-Met-Glu-Gly-Lys-Lys
SEQ ID No. 14: Glu-Lys-Gly-Pro-Arg-Cys-Leu-Thr-Arg-Tyr-Tyr-Ser-Ser-Phe-Val-Asn-Met-Glu-Gly-Lys-Glu
SEQ ID No. 15: Glu-Lys-Gly-Pro-Arg-Cys-Leu-Thr-Arg-Tyr-Tyr-Ser-Ser-Phe-Val-Asn-Met-Glu-Gly-Glu-Lys
SEQ ID No. 16: Glu-Lys-Gly-Pro-Arg-Cys-Leu-Thr-Arg-Tyr-Tyr-Ser-Ser-Phe-Val-Asn-Met-Glu-Gly-Glu-Glu
SEQ ID No. 17: Lys-Lys-Gly-Asp-Asn-Ile-Met-Val-Thr-Phe-Arg-Asn-Gln-Ala-Ser-Arg-Pro-Tyr-Gly-Lys-Lys
SEQ ID No. 18: Lys-Lys-Gly-Asp-Asn-Ile-Met-Val-Thr-Phe-Arg-Asn-Gln-Ala-Ser-Arg-Pro-Tyr-Gly-Lys-Glu
SEQ ID No. 19: Lys-Lys-Gly-Asp-Asn-Ile-Met-Val-Thr-Phe-Arg-Asn-Gln-Ala-Ser-Arg-Pro-Tyr-Gly-Glu-Lys
SEQ ID No. 20: Lys-Lys-Gly-Asp-Asn-Ile-Met-Val-Thr-Phe-Arg-Asn-Gln-Ala-Ser-Arg-Pro-Tyr-Gly-Glu-Glu
SEQ ID No. 21: Glu-Glu-Gly-Asp-Asn-Ile-Met-Val-Thr-Phe-Arg-Asn-Gln-Ala-Ser-Arg-Pro-Tyr-Gly-Lys-Lys
SEQ ID No. 22: Glu-Glu-Gly-Asp-Asn-Ile-Met-Val-Thr-Phe-Arg-Asn-Gln-Ala-Ser-Arg-Pro-Tyr-Gly-Lys-Glu
SEQ ID No. 23: Glu-Glu-Gly-Asp-Asn-Ile-Met-Val-Thr-Phe-Arg-Asn-Gln-Ala-Ser-Arg-Pro-Tyr-Gly-Glu-Lys
SEQ ID No. 24: Glu-Glu-Gly-Asp-Asn-Ile-Met-Val-Thr-Phe-Arg-Asn-Gln-Ala-Ser-Arg-Pro-Tyr-Gly-Glu-Glu
SEQ ID No. 25: Lys-Glu-Gly-Asp-Asn-Ile-Met-Val-Thr-Phe-Arg-Asn-Gln-Ala-Ser-Arg-Pro-Tyr-Gly-Lys-Lys
SEQ ID No. 26: Lys-Glu-Gly-Asp-Asn-Ile-Met-Val-Thr-Phe-Arg-Asn-Gln-Ala-Ser-Arg-Pro-Tyr-Gly-Lys-Glu
SEQ ID No. 27: Lys-Glu-Gly-Asp-Asn-Ile-Met-Val-Thr-Phe-Arg-Asn-Gln-Ala-Ser-Arg-Pro-Tyr-Gly-Glu-Lys
SEQ ID No. 28: Lys-Glu-Gly-Asp-Asn-Ile-Met-Val-Thr-Phe-Arg-Asn-Gln-Ala-Ser-Arg-Pro-Tyr-Gly-Glu-Glu
SEQ ID No. 29: Glu-Lys-Gly-Asp-Asn-Ile-Met-Val-Thr-Phe-Arg-Asn-Gln-Ala-Ser-Arg-Pro-Tyr-Gly-Lys-Lys
SEQ ID No. 30: Glu-Lys-Gly-Asp-Asn-Ile-Met-Val-Thr-Phe-Arg-Asn-Gln-Ala-Ser-Arg-Pro-Tyr-Gly-Lys-Glu
SEQ ID No. 31: Glu-Lys-Gly-Asp-Asn-Ile-Met-Val-Thr-Phe-Arg-Asn-Gln-Ala-Ser-Arg-Pro-Tyr-Gly-Glu-Lys.
SEQ ID No. 32: Glu-Lys-Gly-Asp-Asn-Ile-Met-Val-Thr-Phe-Arg-Asn-Gln-Ala-Ser-Arg-Pro-Tyr-Gly-Glu-Glu.

The term "has" or "having" is intended to mean that the peptide consists of the given amino acid sequence.

The present invention provides a peptide which comprises the sequence DNIMVTFRNQASRPY and has one of the following sequences: SEQ ID No. 17, 18, 19, 25, 26, 27, 29, 30 or 31.

The peptide may have one of the following sequences: SEQ ID No. 17, 18, 25 or 26.

The peptide may have SEQ ID No. 17 or 18.

The peptide may have SEQ ID No.17.

The present invention also provides a peptide which comprises the sequence PRCLTRYYSSFVNME and has one of the following sequences: SEQ ID No. 1, 2, 3, 5, 7, 9, 10, 11 or 13.

The peptide may have one of the following sequences: SEQ ID No. 1, 2, 9 or 10.

The peptide may have SEQ ID No. 1 or 2.

The peptide may have SEQ ID No.1.

### FACTOR VIII

The sequences DNIMVTFRNQASRPY and PRCLTRYYSSFVNME which form the central portion of the peptides of the invention are both derivable from factor VIII.

Factor VIII participates in the intrinsic pathway of blood coagulation; factor VIII is a cofactor for factor IXa which, in the presence of Ca+2 and phospholipids, converts factor X to the activated form Xa.

The factor VIII gene produces two alternatively spliced transcripts. Transcript variant 1 encodes a large glycoprotein, isoform a, which circulates in plasma and associates with von Willebrand factor in a noncovalent complex. This protein undergoes multiple cleavage events. Transcript variant 2 encodes a putative small protein, isoform b, which consists primarily of the phospholipid binding domain of factor Vlllc. This binding domain is essential for coagulant activity.

The complete 186,000 base-pair sequence of the human factor VIII gene was elucidated in the mid 1980s (Gitschier et al (1984) Nature 312 326-330). At the same time, DNA clones encoding the complete 2351 amino acid sequence were used to produce biologically active factor VIII in cultured mammalian cells (Wood et al (1984) Nature 312:330-337). The complete 2,351 amino acid sequence for human factor VIII is given in SEQ ID No. 33.

### SOLUBILITY

The peptide of the first embodiment of the invention is a modified form of one of the following peptides:

| |
|---|
| DNIMVTFRNQASRPY |
| PRCLTRYYSSFVNME |

These peptides have already been shown to act as apitopes and be tolerogenic *in vivo* (See examples and International patent application No PCT/GB2008/003996).

It has since come to light that solubility is an important consideration in peptide-mediated tolerance induction.

The present inventors have found that solubility may be improved by incorporation of a Glycine spacer at both ends, followed by combinations of two additional amino acids which may be Lysine (K) and/or Glutamic acid (E) at both N and C termini. The possible combination at a given terminus is therefore KK, KE, EK or EE.

The modified peptides of the present invention therefore have 6 additional amino acids (3 at each end) than the parent DNIVM and PRCLT peptides.

The peptides of the present invention have the general formula:
XXGDNIMVTFRNQASRPYGXX or
XXGPRCLTRYYSSFVNMEGXX
wherein X is either Lysine or Glutamic acid.

The modified peptide may be more soluble that the parent (unmodified) peptide. The modified peptide may have 2, 3, 4, or 5-fold greater solubility than the parent peptide. The peptide may be soluble at concentrations of up to 0.5 mg/ml, 1 mg/ml, or 5 mg/ml.

### TOLERANCE

T cell epitopes play a central role in the adaptive immune response to any antigen, whether self or foreign. The central role played by T cell epitopes in hypersensitivity diseases (which include allergy, autoimmune diseases and transplant rejection) has been demonstrated through the use of experimental models. It is possible to induce inflammatory or allergic diseases by injection of synthetic peptides (based on the structure of T cell epitopes) in combination with adjuvant.

By contrast, it has been shown to be possible to induce immunological tolerance towards particular antigens by administration of peptide epitopes in soluble form. Administration of soluble peptide antigens has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251-1261); and experimental models of arthritis, diabetes, and uveoretinitis (reviewed in Anderton and Wraith (1998) as above). This has also been demonstrated as a means of treating an ongoing disease in EAE (Anderton and Wraith (1998) as above).

Tolerance is the failure to respond to an antigen. Tolerance to self-antigens is an essential feature of the immune system, when this is lost, autoimmune disease can result. The adaptive immune system must maintain the capacity to respond to an enormous variety of infectious agents while avoiding autoimmune attack of the self-antigens contained within its own tissues. This is controlled to a large extent by the sensitivity of immature T lymphocytes to apoptotic cell death in the thymus (central tolerance). However, not all self-antigens are detected in the thymus, so death of self-reactive thymocytes remains incomplete. There are thus also mechanisms by which tolerance may be acquired by mature self-reactive T lymphocytes in the peripheral tissues (peripheral tolerance). A review of the mechanisms of central and peripheral tolerance is given in Anderton et al (1999) (Immunological Reviews 169:123-137).

In haemophilia A, patients have a defect in the factor VIII gene. This means that factor VIII is not recognised as a "self" antigen by the immune system. When factor VIII is administered during coagulation factor replacement therapy, therefore, an alloimmune response is generated to the foreign protein, leading to the production of FVIII inhibitor antibodies.

The peptides of the present invention are capable of inducing tolerance to factor VIII such that when FVIII is administered prophylactically, it does not induce an immune response and FVIII inhibitors do not develop; and when it is administered therapeutically, ongoing FVIII inhibitor production is reduced or inhibited.

Acquired haemophilia is an autoimmune disease in which tolerance to factor VIII breaks down. In this case, peptides of the present invention may be administered to reinstate tolerance to this self-protein and curtail the pathogenic immune response.

Tolerance may result from or be characterised by the induction of anergy in at least a portion of CD4+ T cells. In order to activate a T cell, a peptide must associate with a "professional" APC capable of delivering two signals to T cells. The first signal (signal 1) is delivered by the MHC-peptide complex on the cell surface of the APC and is received by the T cell via the TCR. The second signal (signal 2) is delivered by costimulatory molecules on the surface of the APC, such as CD80 and CD86, and received by CD28 on the surface of the T cell. It is thought that when a T cell receives signal 1 in the absence of signal 2, it is not activated and, in fact, becomes anergic. Anergic T cells are refractory to subsequent antigenic challenge, and may be capable of suppressing other immune responses. Anergic T cells are thought to be involved in mediating T cell tolerance.

Without wishing to be bound by theory, the present inventors predict that peptides which require processing before they can be presented in conjunction with MHC molecules do not induce tolerance because they have to be handled by mature antigen presenting cells. Mature antigen presenting cells (such as macrophages, B cells and dendritic cells) are capable of antigen processing, but also of delivering both signals 1 and 2 to a T cell, leading to T cell activation. Apitopes, on the other hand, will be able to bind class II MHC on immature APC. Thus they will be presented to T cells without costimulation, leading to T cell anergy and tolerance.

Of course, apitopes are also capable of binding to MHC molecules at the cell surface of mature APC. However, the immune system contains a greater abundance of immature than mature APC (it has been suggested that less than 10% of dendritic cells are activated, Summers et al. (2001) Am. J. Pathol. 159: 285-295). The default position to an apitope will therefore be anergy/tolerance, rather than activation.

The induction of tolerance to FVIII can be monitored *in vivo* by looking for a reduction in the level of:
(i) FVIII inhibitory antibodies:
(ii) CD4+ T cells specific for FVIII
(iii) B cells capable of secreting FVIII inhibitory antibodies
by techniques known in the art.

The induction of tolerance can therefore also be monitored by various techniques including:
(a) the induction of anergy in CD4+ T cells (which can be detected by subsequent challenge with FVIII *in vitro*);
(b) changes in the CD4+ T cell population, including
   (i) reduction in proliferation;
   (ii) down-regulation in the production of IL-2, IFN-γ and IL-4; and
   (iii) increase in the production of IL-10.

As used herein, the term "tolerogenic" means capable of inducing tolerance.

### COMPOSITION

The present invention also relates to a composition, such as a pharmaceutical composition comprising one or more peptide(s) according to the invention.

The composition may comprise a modified form of the peptide DNIMVTFRNQASRPY and a modified form of the peptide PRCLTRYYSSFVNME.

The peptide may comprise a plurality of peptides, for example, two, three, four, five or six peptides.

The composition of the present invention may be for prophylactic or therapeutic use.

When administered for prophylactic use, the composition may reduce or prevent the generation of an immune response to FVIII. The level of immune response is less than would have been obtained in the patient had not been treated with the composition. The term "reduce" indicates that a partial reduction in immune response is observed, such as a 50%, 70%, 80% or 90% reduction in the response that would have been observed in the patient had not been treated with the composition (or in the response observed in an untreated patient over the same time-frame). The term "prevent" indicates that no appreciable immune response to FVIII is observed.

When administered for therapeutic use, the composition may suppress an already ongoing immune response to FVIII. The term "suppress" indicates a reduction in the level of an on-going immune response, compared to the level before peptide treatment, or the level which would have been observed at the same time point had the treatment not been given.

Treatment with the composition of the present invention may cause a reduction in levels of any or all of the following:
(i) FVIII inhibitory antibodies:
(ii) CD4+ T cells specific for FVIII
(iii) B cells secreting FVIII inhibitory antibodies.

Detection of all these factors can be carried out by techniques known in the art, such as ELISA, Flow cytometry, chromogenic clotting assay etc.

Treatment with the composition of the present invention may also or alternatively cause anergy in CD4+ T cells specific for FVIII. Anergy can be detected by for example subsequent challenge with FVIII *in vitro.*

It is important to bear in mind that not all immune responses to FVIII are pathogenic. Non-inhibitory anti-FVIII antibodies may be found in haemophilia patients without inhibitors (Moreau et al (2000) Blood 95:3435-41) and approximately 15% of healthy blood donors (Algiman *et al* (1992) 89:3795-9).

FVIII inhibitors may be detected by the Nijmegen modification of the clotting Bethesda assay, in which the ability of the patient's plasma to inactivate FVIII in normal plasma is tested. A Bethesda unit is defined as the amount of antibody that neutralizes 50% of plasma FVIII activity, and titres of 0.6BU or greater suggest the presence of antibody.

Inhibitors are generally classified as low titre if the level is <5 BU and high titre if ≥ 5 BU.

The level of circulating FVIII inhibitory antibodies may be reduced to 90%, 75%, 50%, 20%, 10% 5% of the level of antibodies which would have been observed had the patient not received treatment.

The level of circulating FVIII inhibitory antibodies may be reduced to 5, 4, 3, 2, 1 or 0.5 BU.

The peptides and composition of the invention may increase the amount or proportion of therapeutically administered FVIII which is available to aid clotting in a patient. This is due to the reduction in FVIII inhibitors which may effectively remove a proportion of FVIII from exerting its therapeutic function. The peptide or composition of the invention may increase the amount of available FVIII by, for example, 10%, 25%, 50% 75% or 100%.

The peptides and composition of the invention may thus reduce the amount of FVIII which needs to be administered to aid clotting in a patient.

### FORMULATION

The composition may by prepared as an injectable, either as liquid solution or suspension; solid form suitable for solution in, or suspension in, liquid prior to injection may also be prepared. The preparation may also be emulsified, or the peptides encapsulated in liposomes. The active ingredients may be mixed with excipients which are pharmaceutically acceptable and compatible with the active ingredient. Suitable excipients are, for example, water, saline (for example, phosphate-buffered saline), dextrose, glycerol, ethanol, or the like and combinations thereof.

In addition, if desired, the composition may contain minor amounts of auxiliary substances such as wetting or emulsifying agents and/or pH buffering agents. Buffering salts include phosphate, citrate, acetate, hydrochloric acid and/or sodium hydroxide may be used for pH adjustment. For stabilisation, disaccharides may be used such as sucrose or trehalose.

If the composition comprises a plurality of peptides, the relative ratio of the peptides may be approximately equal. Alternatively the relative ratios of each peptide may be altered, for example, to focus the tolerogenic response on a particular sub-set of autoreactive T-cells or if it is found that one peptide works better than the others in particular HLA types.

After formulation, the composition may be incorporated into a sterile container which is then sealed and stored at a low temperature, for example 4°C, or it may be freeze-dried.

Conveniently the composition is prepared as a lyophilized (freeze dried) powder. Lyophilisation permits long-term storage in a stabilised form. Lyophilisation procedures are well known in the art, see for example http://www.devicelink.com/ivdt/archive/97/01/006.html. Bulking agents are commonly used prior to freeze-drying, such as mannitol, dextran or glycine.

The composition may be administered in a convenient manner such as by the oral, intravenous (where water soluble), intramuscular, subcutaneous, sublingual, intranasal, intradermal or suppository routes or implanting (e.g. using slow release molecules).

The composition may advantageously be administered via intranasal, subcutaneous or intradermal routes.

The peptide and composition of the invention may be used to treat a human subject. The subject may have haemophilia A, in particular severe haemophilia A. The subject may be genetically deficient in FVIII. The subject may have acquired haemophilia. The subject may have inhibitory anti-FVIII antibodies.

The subject may be undergoing or about to undergo coagulant replacement therapy with FVIII.

The subject may be undergoing or about to undergo therapy with by-passing agents [with or without coagulant replacement therapy with FVIII].

The subject may be undergoing or about to undergo gene therapy with the FVIII gene.

The subject may be an HLA-haplotype which is associated with a predisposition to develop inhibitory anti-FVIII alloantibodies or autoantibodies. The subject may express HLA-DR2. Methods for determining the HLA haplotype of an individual are known in the art.

Typically, a physician will determine the actual dosage which will be most suitable for an individual subject and it will vary with the age, weight and response of the particular patient.

In a preferred embodiment a "dose escalation" protocol may be followed, where a plurality of doses is given to the patient in ascending concentrations. Such an approach has been used, for example, for phospholipase A2 peptides in immunotherapeutic applications against bee venom allergy (Müller et al (1998) J. Allergy Clin Immunol. 101:747-754 and Akdis et al (1998) J. Clin. Invest. 102:98-106).

### KITS

Conveniently, if the composition comprises a plurality of peptides, they may be administered together, in the form of a mixed composition or cocktail. However, there may be circumstances in which it is preferable to provide the peptides separately in the form of a kit, for simultaneous, separate, sequential or combined administration.

The kit may also comprise mixing and/or administration means (for example a vapouriser for intranasal administration; or a syringe and needle for subcutaneous/intradermal dosing). The kit may also comprise instructions for use.

The pharmaceutical composition or kit of the invention may be used to treat and/or prevent a disease.

In particular, the composition/kit may be used to treat and/or prevent anti-FVIII inhibitor formation in haemophilia A or acquired haemophilia patients. The composition/kit may be used to treat haemophilia impaired by the presence of neutralising FVIII antibodies.

### HAEMOPHILIA A

Haemophilia A (classic haemophilia), is caused by the deficiency of Factor FVIII.

Haemophilia A has an estimated incidence of 1 in 10,000 males, while haemophilia B is estimated to occur in one in 40,000 males. Approximately 1 woman in 5,000 is a carrier for haemophilia A, and 1 in 20,000 is a carrier of haemophilia B. Haemophilia is typically divided into three classes: severe, moderate and mild, based on the level of clotting factor in the blood. In severe haemophilia, there is less than 1 percent of normal clotting factor. The degree of severity tends to be consistent from generation to generation.

Contrary to popular belief, minor cuts and wounds do not usually present a threat to haemophiliacs. Rather, the greatest danger comes from spontaneous bleeding that may occur in joints and muscles. This is most prone to occur during years of rapid growth, typically between the ages of 5 and 15 years.

Repeated spontaneous bleeding in joints may cause arthritis, and adjacent muscles become weakened. Pressure on nerves caused by the accumulation of blood may result in pain, numbness, and temporary inability to move the affected area.

Haemophilia A is usually diagnosed with a blood test to determine the effectiveness of clotting and to investigate whether the levels of clotting factors are abnormal.

The development of purified clotting factors in the 1970s, isolated from donated blood, significantly improved the long-term outlook for haemophiliacs. Mild to moderate haemophiliacs can use treatment with FVII on an *ad hoc* basis, whereas severe haemophiliacs may require regular, indefinite treatment.

Previously, patients were given factor VIII concentrates pooled from thousands of plasma donations. This leads to significant problems of contamination with viral pathogens, particularly the human immunodeficiency virus and the hepatitis viruses. Monoclonal antibody purification techniques, heat inactivation, and virucidal detergent treatments have rendered plasma-derived concentrates relatively safe.

Recombinant DNA technology has now provided a series of synthetic products, such as Recombinate™ and Kogenate™. Kogenate is made using baby hamster kidney cells expressing human factor VIII. The resulting factor is highly purified, eliminating any possibility of transmission of virus from plasma.

The peptide or composition of the present invention may be administered before and/or during factor VIII replacement therapy.

Haemophilia A is an ideal disease target for gene therapy since i) it is caused by a mutations in a single identified gene, ii) a slight increase in clotting factor levels *in vivo* can convert severe haemophilia into milder disease, and iii) current replacement therapies are considered suboptimal. Also, there is a wide range of safety if there is an "overshoot" of desired level of coagulation activity.

Unfortunately, to date the promise of gene therapy as a cure for haemophilia has not been realized, primarily because of difficulties in finding a gene delivery system which is sufficiently non-immunogenic to allow for long term expression of the clotting factor.

The peptides of the present invention is also suitable for tolerising a subject prior to gene therapy with factor VIII and/or managing FVIII inhibitor formation in a patient following gene therapy.

### ACQUIRED HAEMOPHILIA

Acquired haemophilia is characterised by the presence of autoantibody inhibitors against FVIII in individuals with previously normal coagulation. It is a rare condition, with an estimated incidence of 1-3 per million population per year. The mortality rate associated with acquired autoantibody inhibitors approaches 25% versus the substantially lower risk of death in those with alloantibodies.

Compared to alloantibody inhibitor patients, acquired haemophilia is characterized by: (1) a more severe bleeding pattern; (2) higher incidence in older population; (3) occurrence in conjunction with identifiable underlying autoimmune diseases, lymphoproliferative or solid tumor malignancies, pregnancy, and use of certain antibiotics such as penicillin and sulfonamides in approximately 50% of cases; and (4) in vitro inhibitor activity that follow a type II pharmacokinetic pattern with incomplete neutralization of the targeted clotting factor activity by the autoantibody, typically resulting in residual factor VIII levels ranging between 2%-18% in patient plasma.

The peptide or composition of the present invention may be administered to a patient with acquired haemophilia, or to a patient believed to be at risk of developing acquired haemophilia due to, for example:
i) imminent treatment with, for example penicillin or a sulfonamide
ii) progression of a tumour or other malignancy
iii) imminent or early pregnancy.

The invention will now be further described by way of Examples, which are meant to serve to assist one of ordinary skill in the art in carrying out the invention and are not intended in any way to limit the scope of the invention.

### EXAMPLES

### Example 1 - Investigating the solubility of FVIII-derived peptides.

A total of 32 peptides were tested: 16 of which were based on the FVIII peptide PRCLTRYYSSFVNME ("PRCLT"); and 16 of which were based on the FVIII peptide DNIMVTFRNQASRPY ("DNIMV"). The peptides are summarised in Tables 1 and 2 below.

**Table 1 - PRCLT-derived peptides**

| Peptide No | Sequence | SEQ ID No. |
|---|---|---|
| 1 | Lys-Lys-Gly-Pro-Arg-Cys-Leu-Thr-Arg-Tyr-Tyr-Ser-Ser- Phe-Val-Asn-Met-Glu-Gly-Lys-Lys | 1 |
| 2 | Lys-Lys-Gly-Pro-Arg-Cys-Leu-Thr-Arg-Tyr-Tyr-Ser-Ser- Phe-Val-Asn-Met-Glu-Gly-Lys-Glu | 2 |
| 3 | Lys-Lys-Gly-Pro-Arg-Cys-Leu-Thr-Arg-Tyr-Tyr-Ser-Ser- Phe-Val-Asn-Met-Glu-Gly-Glu-Lys | 3 |
| 4 | Lys-Lys-Gly-Pro-Arg-Cys-Leu-Thr-Arg-Tyr-Tyr-Ser-Ser- Phe-Val-Asn-Met-Glu-Gly-Glu-Glu | 4 |
| 5 | Glu-Glu-Gly-Pro-Arg-Cys-Leu-Thr-Arg-Tyr-Tyr-Ser-Ser- Phe-Val-Asn-Met-Glu-Gly-Lys-Lys | 5 |
| 6 | Glu-Glu-Gly-Pro-Arg-Cys-Leu-Thr-Arg-Tyr-Tyr-Ser-Ser- Phe-Val-Asn-Met-Glu-Gly-Lys-Glu | 6 |
| 7 | Glu-Glu-Gly-Pro-Arg-Cys-Leu-Thr-Arg-Tyr-Tyr-Ser-Ser- Phe-Val-Asn-Met-Glu-Gly-Glu-Lys | 7 |
| 8 | Glu-Glu-Gly-Pro-Arg-Cys-Leu-Thr-Arg-Tyr-Tyr-Ser-Ser- Phe-Val-Asn-Met-Glu-Gly-Glu-Glu | 8 |
| 9 | Lys-Glu-Gly-Pro-Arg-Cys-Leu-Thr-Arg-Tyr-Tyr-Ser-Ser- Phe-Val-Asn-Met-Glu-Gly-Lys-Lys | 9 |
| 10 | Lys-    Glu-Gly-Pro-Arg-Cys-Leu-Thr-Arg-Tyr-Tyr-Ser-Ser- Phe-Val-Asn-Met-Glu-Gly-Lys-Glu | 10 |
| 11 | Lys-    Glu-Gly-Pro-Arg-Cys-Leu-Thr-Arg-Tyr-Tyr-Ser-Ser- Phe-Val-Asn-Met-Glu-Gly-Glu-Lys | 11 |
| 12 | Lys-    Glu-Gly-Pro-Arg-Cys-Leu-Thr-Arg-Tyr-Tyr-Ser-Ser- Phe-Val-Asn-Met-Glu-Gly-Glu-Glu | 12 |
| 13 | Glu-Lys-Gly-Pro-Arg-Cys-Leu-Thr-Arg-Tyr-Tyr-Ser-Ser- Phe-Val-Asn-Met-Glu-Gly-Lys-Lys | 13 |
| 14 | Glu-Lys-Gly-Pro-Arg-Cys-Leu-Thr-Arg-Tyr-Tyr-Ser-Ser- Phe-Val-Asn-Met-Glu-Gly-Lys-Glu | 14 |
| 15 | Glu-Lys-Gly-Pro-Arg-Cys-Leu-Thr-Arg-Tyr-Tyr-Ser-Ser- Phe-Val-Asn-Met-Glu-Gly-Glu-Lys | 15 |
| 16 | Glu-Lys-Gly-Pro-Arg-Cys-Leu-Thr-Arg-Tyr-Tyr-Ser-Ser- Phe-Val-Asn-Met-Glu-Gly-Glu-Glu | 16 |

**Table 2 - DNIMV-derived peptides**

| Peptide No | Sequence | SEQ ID No. |
|---|---|---|
| 17 | Lys-Lys-Gly-Asp-Asn-Ile-Met-Val-Thr-Phe-Arg-Asn-Gln-Ala- Ser-Arg-Pro-Tyr-Gly-Lys-Lys | 17 |
| 18 | Lys-Lys-Gly-Asp-Asn-Ile-Met-Val-Thr-Phe-Arg-Asn-Gln-Ala- | 18 |
| | Ser-Arg-Pro-Tyr-Gly-Lys-Glu | |
| 19 | Lys-Lys-Gly-Asp-Asn-Ile-Met-Val-Thr-Phe-Arg-Asn-Gln-Ala- Ser-Arg-Pro-Tyr-Gly-Glu-Lys | 19 |
| 20 | Lys-Lys-Gly-Asp-Asn-Ile-Met-Val-Thr-Phe-Arg-Asn-Gln-Ala- Ser-Arg-Pro-Tyr-Gly-Glu-Glu | 20 |
| 21 | Glu-Glu-Gly-Asp-Asn-Ile-Met-Val-Thr-Phe-Arg-Asn-Gln-Ala- Ser-Arg-Pro-Tyr-Gly-Lys-Lys | 21 |
| 22 | Glu-Glu-Gly-Asp-Asn-Ile-Met-Val-Thr-Phe-Arg-Asn-Gln-Ala- Ser-Arg-Pro-Tyr-Gly-Lys-Glu | 22 |
| 23 | Glu-Glu-Gly-Asp-Asn-Ile-Met-Val-Thr-Phe-Arg-Asn-Gln-Ala- Ser-Arg-Pro-Tyr-Gly-Glu-Lys | 23 |
| 24 | Glu-Glu-Gly-Asp-Asn-Ile-Met-Val-Thr-Phe-Arg-Asn-Gln-Ala- Ser-Arg-Pro-Tyr-Gly-Glu-Glu | 24 |
| 25 | Lys-Glu-Gly-Asp-Asn-Ile-Met-Val-Thr-Phe-Arg-Asn-Gln-Ala- Ser-Arg-Pro-Tyr-Gly-Lys-Lys | 25 |
| 26 | Lys-    Glu-Gly-Asp-Asn-Ile-Met-Val-Thr-Phe-Arg-Asn-Gln-Ala- Ser-Arg-Pro-Tyr-Gly-Lys-Glu | 26 |
| 27 | Lys-    Glu-Gly-Asp-Asn-Ile-Met-Val-Thr-Phe-Arg-Asn-Gln-Ala- Ser-Arg-Pro-Tyr-Gly-Glu-Lys | 27 |
| 28 | Lys-    Glu-Gly-Asp-Asn-Ile-Met-Val-Thr-Phe-Arg-Asn-Gln-Ala- Ser-Arg-Pro-Tyr-Gly-Glu-Glu | 28 |
| 29 | Glu-Lys-Gly-Asp-Asn-Ile-Met-Val-Thr-Phe-Arg-Asn-Gln-Ala- Ser-Arg-Pro-Tyr-Gly-Lys-Lys | 29 |
| 30 | Glu-Lys-Gly-Asp-Asn-Ile-Met-Val-Thr-Phe-Arg-Asn-Gln-Ala- Ser-Arg-Pro-Tyr-Gly-Lys-Glu | 30 |
| 31 | Glu-Lys-Gly-Asp-Asn-Ile-Met-Val-Thr-Phe-Arg-Asn-Gln-Ala- Ser-Arg-Pro-Tyr-Gly-Glu-Lys | 31 |
| 32 | Glu-Lys-Gly-Asp-Asn-Ile-Met-Val-Thr-Phe-Arg-Asn-Gln-Ala- Ser-Arg-Pro-Tyr-Gly-Glu-Glu | 32 |

A stock solution of each test peptide of 20mg/mL was prepared in DMSO. A stock solution of the control peptide, an MBP-derived peptide known as 4Y was also prepared in DMSO. Peptide 4Y has the sequence Ac-ASQYRPSQR. It is structurally dissimilar to the test peptides but is known to be sufficiently soluble for application as a tolerising antigen.

Sequential dilutions of a 40µL volume of peptide solution were made by adding water in a 96-well plate to give concentrations of 10, 5, 2.5 and 1.25 mg/mL

The plates were left at room temperature for 1 hour to allow any precipitate to form and then centrifuged at 14,800rpm for 10 minutes to separate precipitated/colloidal peptide from truly dissolved solute.

A 2µL sample from the very top of each tube was removed for analysis. Absorbance was measured at a wavelength of 280nm and concentration (mg/mL) was calculated.

The results are shown in Figure 1. Some tagged peptides demonstrated a solubility equivalent to 4Y, even at high concentration. Peptides coded green were as soluble as control peptide 4Y, peptides coded red were poorly soluble, and one peptide coded amber had intermediate solubility.

### Example 2 - Direct dissolution of tagged peptides in aqueous solvent at clinically relevant concentration

For each test peptide and the parent peptides (PRCLT and DNIMV) two tubes were prepared at 4mg/mL in either DMSO (control) or PBS.

Each tube was spun at full speed in a mini-centrifuge for 5 minutes. A 10µL sample was removed from the top of each solution and the molecular weight and coefficient of absorption was recorded for each peptide.

The absorbance of the supernatant was measured using NanoDrop and the concentration calculated. The actual concentration was compared to the expected value of 4mg/mL and the results are shown in Figure 2. In this Figure, greater disparity between the actual and expected concentration corresponds to lower relative solubility.

As shown in Figure 2, the tagged peptides SEQ ID No. 1, 2, 9, 10, 17, 18, 25 and 26 all had improved solubility in PBS compared to the parent peptides.

### Example 3 - The tagged peptides act as apitopes

HLA-DR2 transgenic mice were primed with the parent peptides PRCLT or DNIMV. Spleen and lymph nodes were then harvested, and after CD4 purification, cells were restimulated with 1ug/ml human recombinant FVIII. After fusion with BW5147 cells, the resulting clones were expanded and 'screened' for specificity to DNIMV or PRCLT. This is performed by incubating hybridoma cells with 5x10⁴ DR2-positive antigen presenting cells (MGAR cell line) and 10ug/ml PRCLT/DNIMV or media for 48 hours. The supernatants were then analysed for IL-2 production by ELISA.

Clones which produced IL-2 specifically when incubated with peptide were expanded and screened again with FVIII (at 1ug/ml).

Clones which produced IL-2 in response to both FVIII and peptide were then used to assess whether the modified DNIMV and PRCLT peptides are apitopes i.e. whether they are capable of binding to an MHC molecule and being presented to a T cell by both fixed and fresh APC.

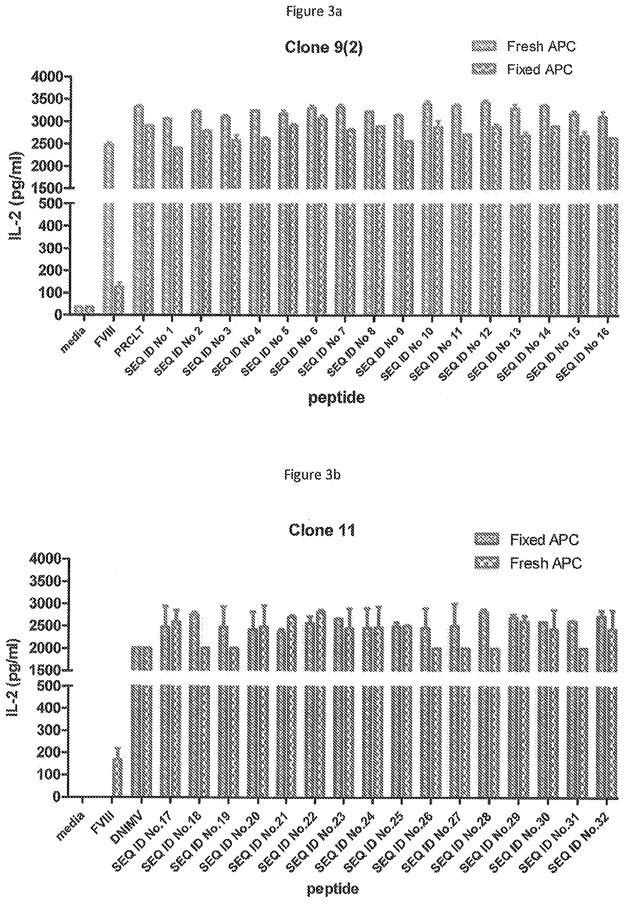

5x10⁴ hybridoma cells were incubated with 5x10⁴ fresh or fixed MGAR cells, and 10ug/ml of peptide, 1ug/ml FVIII or media for 48 hours. Supernatants were then analysed for IL-2 using ELISA. The results are shown for PRCLT in Figure 3a and for DNIMV in Figure 3b.

All of the tagged peptides were capable of being presented by fixed antigen presenting cells, indicating that they are all apitopes like the parent peptides PRCLT and DNIMV.

### Example 4: Ex vivo T cell tolerance

To determine if modified apitopes were capable of regulating T cell responses to parent peptides, male HLA-DR2 transgenic mice were treated with 3x100µg peptide (peptides SEQ ID No. 1, 2, 17 or 18) or PBS as a control, and then immunised with 100µg of each of the parent peptides (PRCLT and DNIMV) in complete Freund's Adjuvant. Ten days later, draining lymph nodes and spleens were collected and stimulated *in vitro* with either PRCLT or DNIMV. The results are shown in Figures 4 (PRCLT) and 5 (DNIMV). Treatment with modified peptides SEQ ID No. 1 or SEQ ID No. 2 reduces T cell responses to PRCLT and treatment with modified peptides SEQ ID No. 17 or SEQ ID No. 18 reduces T cell responses to DNIMV. Thus the modified peptides are able to reduce an immune response from mice immunised with the parent peptide.

### Example 5: Ex vivo T cell tolerance

In order to determine if the effect of modified apitopes on the regulation of T cell responses to parental peptides extended to regulating responses to FVIII, female HLA-DR2 transgenic mice were treated with 3x100µg peptide (peptides SEQ ID No. 1 or SEQ ID No. 17), and then primed with 100µg of each of the parent peptides (PRCLT and DNIMV) in complete Freund's Adjuvant. Ten days later, draining lymph nodes were collected and stimulated in vitro with either PRCLT, DNIMV or recombinant FVIII. The results are shown in Figure 6. Peptide SEQ ID no 1 significantly (p<0.01) reduced the T cell response to the PRCLT peptide. Peptide SEQ ID no 17 significantly (p<0.02) reduced the T cell response to DNIMV.

Peptide SEQ ID No.1 regulated responses to PRCLT and peptide SEQ ID No.17 regulated responses to DNIMV. There was also some reduction, which was non-significant, in response to FVIII with individual peptides. To enhance efficacy of the peptides against FVIII immune responses a combination of modified DNIMV and modified PRCLT were used in further experiments.

### Example 6: Antibody generation prevention with a dose escalation of a combination of PRCLT and DNIMV modified peptides

Male HLA-DR2 transgenic mice were treated with the combination of peptide SEQ ID No. 1 and peptide SEQ ID No. 17 following a dose escalation protocol. Six injections of the combination were given at the following dose: 0.1; 1.0; 10 and 3x100 µg (for each peptide in the combination).

Animals were then primed weekly with 1µg recombinant human FVIII and a concurrent treatment (3 days after rFVIII immunisation) with a combination of peptides SEQ ID No.1/SEQ ID No.17 (100 µg each) and the generation of anti-FVIII antibodies analysed from blood samples taken after 4 and 8 FVIII immunisations (day 28, Figure 7a; day 56, Figure 7b, respectively). Sera were titrated and analysed by direct ELISA. The antibody titre for unimmunised mice was negative in all cases, whereas anti-FVIII antibody was considered present in the sera of treated mice where binding ratio (using negative sera to calculate the ratio) was greater than 1.9. Results are shown in Figure 7 a and b for day 28 and day 56 of the experiment, respectively.

In addition, neutralizing FVIII antibodies were measured on day 56. FVIII neutralizing antibodies were determined using a chromogenic method. Briefly, samples were mixed with 1 IU/ml FVIII and coagulation was initiated by addition of FIX, FX, Trombin, CaCl₂ and phospholipids. After incubation the amount of generated FXa was determined by addition of the chromogenic substrate S-2760 and the OD signal was measured. The OD signal is proportional to the FVIII activity in the samples and is compared to samples containing a known amount of FVIII and no neutralizing antibodies. The % remaining activity of the test sample was calculated compared to the reference samples and expressed in Bethesda like units and shown in Figure 8. Mice treated with peptides SEQ ID1 and SEQ ID17 had a significantly lower level of neutralizing anti-FVIII antibodies than mice treated with PBS.

### Example 7: Ex vivo T cell tolerance using a dose escalation of a combination of PRCLT and DNIMV modified peptides

Male/female DR2 mice were treated with the combination of peptides SEQ ID No1/SEQ ID No17 following a dose escalation protocol as described in Example 6.

Animals were primed with 100µg PRCLT and 100µg DNIMV in CFA. Ten days later draining lymph nodes cells and splenocytes were isolated and restimulated *in vitro with* recombinant human FVIII.

T cell responses were assessed by proliferation assays (Figure 9). A significant reduction of T cell responses to FVIII were seen across all concentrations of FVIII tested when the mice were treated with the combination of peptides SEQ ID No1 and SEQ ID No17.

### Example 8: Suppression of neutralising antibody generation with a dose escalation of a combination of PRCLT and DNIMV modified peptides in a therapeutic animal model

HLA-DR2 transgenic mice were primed with 3µg rFVIII in CFA and three weeks after the immunisation treated with the combination of peptide SEQ ID No. 1 and peptide SEQ ID No. 17 following a dose escalation protocol. Six injections of the combination were given at the following dose: 0.1; 1.0; 10 and 3x100 µg (for each peptide in the combination). Control animals were treated with a non-related FVIII DR2-binding prostatic acid phosphatase (PAP) 133-152 peptide (sequence as described in patent PCT/US2006/031961, SEQ ID No. 15) using the same dose-escalation protocol. All animals received a boost immunization with 3µg rhFVIII in IFA five weeks after rFVIII immunisation and 4 days after the last round of peptide treatment. Generation of neutralising antibodies was analysed from plasma samples taken 3 weeks after the first FVIII immunisation and prior to any peptide treatment, at week 5 (prior to the FVIII boost immunisation and after the peptide treatment) and during a follow-up period at week 7, 9 and 13. Neutralising FVIII antibody levels in the plasma samples were determined as described in Example 6.

Results in figure 10 show that treatment with peptides SEQ ID No. 1 and SEQ ID No. 17 three weeks after immunisation with FVIII (Week 3) suppress the formation of neutralizing anti-FVIII antibodies significantly after the boost immunization (Week 5) compared to treatment with control peptide.

Although the invention has been described in connection with specific preferred embodiments, it should be understood that the invention as claimed should not be unduly limited to such specific embodiments. Indeed, various modifications of the described modes for carrying out the invention which are obvious to those skilled in molecular immunology or related fields are intended to be within the scope of the following claims.

### SEQ ID No.33

1 mqielstcff lcllrfcfsa trryylgave lswdymqsdl gelpvdarfp prvpksfpfn
61 tsvvykktlf veftdhlfni akprppwmgl lgptiqaevy dtvvitlknm ashpvslhav
121 gvsywkaseg aeyddqtsqr ekeddkvfpg gshtyvwqvl kengpmasdp lcltysylsh
181 vdlvkdlnsg ligallvcre gslakektqt lhkfillfav fdegkswhse tknslmqdrd
241 aasarawpkm htvngyvnrs lpgligchrk svywhvigmg ttpevhsifl eghtflvrnh
301 rqasleispi tfltaqtllm dlgqfllfch isshqhdgme ayvkvdscpe epqlrmknne
361 eaedydddlt dsemdvvrfd ddnspsfiqi rsvakkhpkt wvhyiaaeee dwdyaplvla
421 pddrsyksqy lnngpqrigr kykkvrfmay tdetfktrea iqhesgilgp llygevgdtl
481 liifknqasr pyniyphgit dvrplysrrl pkgvkhlkdf pilpgeifky kwtvtvedgp
541 tksdprcltr yyssfvnmer dlasgligpl licykesvdq rgnqimsdkr nvilfsvfde
601 nrswylteni qrflpnpagv qledpefqas nimhsingyv fdslqlsvcl hevaywyils
661 igaqtdflsv ffsgytfkhk mvyedtltlf pfsgetvfms menpglwilg chnsdfrnrg
721 mtallkvssc dkntgdyyed syedisayll sknnaieprs fsqnsrhpst rqkqfnatti
781 pendiektdp wfahrtpmpk iqnvsssdll mllrqsptph glslsdlqea kyetfsddps
841 pgaidsnnsl semthfrpql hhsgdmvftp esglqlrlne klgttaatel kkldfkvsst
901 snnlistips dnlaagtdnt sslgppsmpv hydsqldttl fgkkssplte sggplslsee
961 nndskllesg lmnsqesswg knvsstesgr lfkgkrahgp alltkdnalf kvsisllktn
1021 ktsnnsatnr kthidgpsll iensps vwqn ilesdtefkk vtplihdrml mdknatalrl
1081 nhmsnkttss knmemvqqkk egpippdaqn pdmsffkmlf lpesarwiqr thgknslnsg
1141 qgpspkqlvs lgpeksvegq nflseknkvv vgkgeftkdv glkemvfpss rnlfltnldn
1201 lhennthnqe kkiqeeiekk etliqenvvl pqihtvtgtk nfmknlflls trqnvegsyd
1261 gayapvlqdf rslndstnrt kkhtahfskk geeenleglg nqtkqiveky acttrispnt
1321 sqqnfvtqrs kralkqfrlp leetelekri ivddtstqws knmkhltpst ltqidyneke
1381 kgaitqspls dcltrshsip qanrsplpia kvssfpsirp iyltrvlfqd nsshlpaasy
1441 rkkdsgvqes shflqgakkn nlslailtle mtgdqrevgs lgtsatnsvt ykkventvlp
1501 kpdlpktsgk vellpkvhiy qkdlfptets ngspghldlv egsllqgteg aikwneanrp
1561 gkvpflrvat essaktpskl ldplawdnhy gtqipkeewk sqekspekta fkkkdtilsl
1621 nacesnhaia ainegqnkpe ievtwakqgr terlcsqnpp vlkrhqreit rttlqsdqee
1681 idyddtisve mkkedfdiyd edenqsprsf qkktrhyfia averlwdygm sssphvlrnr
1741 aqsgsvpqfk kvvfqeftdg sftqplyrge lnehlgllgp yiraevedni mvtfrnqasr
1801 pysfysslis yeedqrqgae prknfvkpne tktyfwkvqh hmaptkdefd ckawayfsdv
1861 dlekdvhsgl igpllvchtn tlnpahgrqv tvqefalfft ifdetkswyf tenmerncra
1921 pcniqmedpt fkenyrfhai ngyimdtlpg lvmaqdqrir wyllsmgsne nihsihfsgh
1981 vftvrkkeey kmalynlypg vfetvemlps kagiwrvecl igehlhagms tlflvysnkc

2041 qtplgmasgh irdfqitasg qygqwapkla rlhysgsina wstkepfswi kvdllapmii
2101 hgiktqgarq kfsslyisqf iimysldgkk wqtyrgnstg tlmvffgnvd ssgikhnifn
2161 ppiiaryirl hpthysirst lrmewmgcdl nscsmplgme skaisdaqit assyftnmfa
2221 twspskarlh lqgrsnawrp qvnnpkewlq vdfqktmkvt gvttqgvksl ltsmyvkefl
2281 isssqdghqw tlffqngkvk vfqgnqdsft pvvnsldppl ltrylrihpq swvhqialrm
2341 evlgceaqdl y

## Claims

1. A peptide comprising the FVIII-derived sequence DNIMVTFRNQASRPY which peptide (a) has the formula
XXGDNIMVTFRNQASRPYGXX
wherein X is either Lysine or Glutamic acid; and
(b) has one of the following sequences:
KKGDNIMVTFRNQASRPYGKK (SEQ ID No. 17)
KKGDNIMVTFRNQASRPYGKE (SEQ ID No. 18)
KKGDNIMVTFRNQASRPYGEK (SEQ ID No. 19)
KEGDNIMVTFRNQASRPYGKK (SEQ ID No. 25)
KEGDNIMVTFRNQASRPYGKE (SEQ ID No. 26)
KEGDNIMVTFRNQASRPYGEK (SEQ ID No. 27)
EKGDNIMVTFRNQASRPYGKK (SEQ ID No. 29)
EKGDNIMVTFRNQASRPYGKE (SEQ ID No. 30) and
EKGDNIMVTFRNQASRPYGEK (SEQ ID No. 31).

2. A peptide comprising the FVIII-derived sequence PRCLTRYYSSFVNME which peptide (a) has the formula
XXGPRCLTRYYSSFVNMEGXX
wherein X is either Lysine or Glutamic acid; and
(b) has one of the following sequences:
KKGPRCLTRYYSSFVNMEGKK (SEQ ID No. 1)
KKGPRCLTRYYSSFVNMEGKE (SEQ ID No. 2)
KKGPRCLTRYYSSFVNMEGEK (SEQ ID No. 3)
EEGPRCLTRYYSSFVNMEGKK (SEQ ID No. 5)
EEGPRCLTRYYSSFVNMEGEK (SEQ ID No. 7)
KEGPRCLTRYYSSFVNMEGKK (SEQ ID No. 9)
KEGPRCLTRYYSSFVNMEGKE (SEQ ID No. 10)
KEGPRCLTRYYSSFVNMEGEK (SEQ ID No. 11) and
EKGPRCLTRYYSSFVNMEGKK (SEQ ID No. 13).

3. A composition comprising a plurality of peptides, including one or more peptide(s) according to claim 1 or 2.

4. A composition according to claim 3, which comprises at least one peptide according to claim 1 and at least one peptide according to claim 2.

5. A composition according to claim 4, which comprises a peptide having SEQ ID No. 1 and a peptide having SEQ ID No. 17

6. A peptide according to claim 1 or 2, or a composition according to any of claims 3 to 5, for use in suppressing or preventing the production of factor VIII inhibitor antibodies *in vivo.*

7. A peptide according to claim 1 or 2, or a composition according to any of claims 3 to 5, for use in a method for treating haemophilia in a subject.

8. A peptide or composition for use according to claim 7, wherein the subject has haemophilia A, and is undergoing, or is about to undergo, factor VIII replacement therapy and/or FVIII by-passing therapy.

9. A peptide or composition for use according to claim 7, wherein the subject has, or is at risk from contracting, acquired haemophilia.

10. A peptide or composition for use according to any of claims 7 to 9, wherein the subject is HLA-DR2.

## Patentansprüche

1. Peptid, umfassend die von FVIII abgeleitete Sequenz DNIMVTFRNQASRPY, wobei das Peptid (a) die Formel
XXGDNIMVTFRNQASRPYGXX
aufweist,
wobei X entweder Lysin oder Glutaminsäure bedeutet; und
(b) eine der folgenden Sequenzen aufweist:
KKGDNIMVTFRNQASRPYGKK (SEQ ID NR. 17)
KKGDNIMVTFRNQASRPYGKE (SEQ ID NR. 18)
KKGDNIMVTFRNQASRPYGEK (SEQ ID NR. 19)
KEGDNIMVTFRNQASRPYGKK (SEQ ID NR. 25)
KEGDNIMVTFRNQASRPYGKE (SEQ ID NR. 26)
KEGDNIMVTFRNQASRPYGEK (SEQ ID NR. 27)
EKGDNIMVTFRNQASRPYGKK (SEQ ID NR. 29)
EKGDNIMVTFRNQASRPYGKE (SEQ ID NR. 30) und
EKGDNIMVTFRNQASRPYGEK (SEQ ID NR. 31).

2. Peptid, umfassend die von FVIII abgeleitete Sequenz PRCLTRYYSSFVNME, wobei das Peptid (a) die Formel
XXGPRCLTRYYSSFVNMEGXX
aufweist,
wobei X entweder Lysin oder Glutaminsäure bedeutet; und
(b) eine der folgenden Sequenzen aufweist:
KKGPRCLTRYYSSFVNMEGKK (SEQ ID NR. 1)
KKGPRCLTRYYSSFVNMEGKE (SEQ ID NR. 2)
KKGPRCLTRYYSSFVNMEGEK (SEQ ID NR. 3)
EEGPRCLTRYYSSFVNMEGKK (SEQ ID NR. 5)
EEGPRCLTRYYSSFVNMEGEK (SEQ ID NR. 7)
KEGPRCLTRYYSSFVNMEGKK (SEQ ID NR. 9)
KEGPRCLTRYYSSFVNMEGKE (SEQ ID NR. 10)
KEGPRCLTRYYSSFVNMEGEK (SEQ ID NR. 11) und
EKGPRCLTRYYSSFVNMEGKK (SEQ ID NR. 13).

3. Zusammensetzung, umfassend eine Vielzahl von Peptiden, einschließlich eines oder mehrerer Peptide nach Anspruch 1 oder 2.

4. Zusammensetzung nach Anspruch 3, die mindestens ein Peptid nach Anspruch 1 und mindestens ein Peptid nach Anspruch 2 umfasst.

5. Zusammensetzung nach Anspruch 4, die ein Peptid mit der SEQ ID NR. 1 und ein Peptid mit der SEQ ID NR. 17 umfasst.

6. Peptid nach Anspruch 1 oder 2, oder Zusammensetzung nach einem der Ansprüche 3 bis 5, zur Verwendung bei der Unterdrückung oder Vorbeugung der Produktion von Faktor-VIII-Inhibitor-Antikörpern *in vivo.*

7. Peptid nach Anspruch 1 oder 2, oder Zusammensetzung nach einem der Ansprüche 3 bis 5, zur Verwendung in einem Verfahren zur Behandlung von Hämophilie in einem Individuum.

8. Peptid oder Zusammensetzung zur Verwendung nach Anspruch 7, wobei das Individuum an Hämophilie A leidet und eine Faktor-VIII-Ersatztherapie und/oder FVIII-Bypass-Therapie macht oder demnächst machen wird.

9. Peptid oder Zusammensetzung zur Verwendung nach Anspruch 7, wobei das Individuum an erworbener Hämophilie leidet oder Gefahr läuft, sich diese zuzuziehen.

10. Peptid oder Zusammensetzung zur Verwendung nach einem der Ansprüche 7 bis 9, wobei das Individuum HLA-DR2 ist.

## Revendications

1. Peptide comprenant la séquence dérivée du FVIII DNIMVTFRNQASRPY lequel peptide (a) a la formule
XXGDNIMVTFRNQASRPYGXX
dans laquelle X est soit une lysine soit un acide glutamique ; et
(b) a l'une des séquences suivantes :
KKGDNIMVTFRNQASRPYGKK (SEQ ID n° 17)
KKGDNIMVTFRNQASRPYGKE (SEQ ID n° 18)
KKGDNIMVTFRNQASRPYGEK (SEQ ID n° 19)
KEGDNIMVTFRNQASRPYGKK (SEQ ID n° 25)
KEGDNIMVTFRNQASRPYGKE (SEQ ID n° 26)
KEGDNIMVTFRNQASRPYGEK (SEQ ID n° 27)
EKGDNIMVTFRNQASRPYGKK (SEQ ID n° 29)
EKGDNIMVTFRNQASRPYGKE (SEQ ID n° 30) et
EKGDNIMVTFRNQASRPYGEK (SEQ ID n°31).

2. Peptide comprenant la séquence dérivée du FVIII PRCLTRYYSSFVNME lequel peptide (a) a la formule
XXGPRCLTRYYSSFVNMEGXX
dans laquelle X est soit une lysine soit un acide glutamique ; et
(b) a l'une des séquences suivante
KKGPRCLTRYYSSFVNMEGKK (SEQ ID n° 1)
KKGPRCLTRYYSSFVNMEGKE (SEQ ID n° 2)
KKGPRCLTRYYSSFVNMEGEK (SEQ ID n° 3)
EEGPRCLTRYYSSFVNMEGKK (SEQ ID n° 5)
EEGPRCLTRYYSSFVNMEGEK (SEQ ID n° 7)
KEGPRCLTRYYSSFVNMEGKK (SEQ ID n° 9)
KEGPRCLTRYYSSFVNMEGKE (SEQ ID n° 10)
KEGPRCLTRYYSSFVNMEGEK (SEQ ID n° 11) et
EKGPRCLTRYYSSFVNMEGKK (SEQ ID n° 13).

3. Composition comprenant une pluralité de peptides, y compris un ou plusieurs peptide(s) selon la revendication 1 ou 2.

4. Composition, selon la revendication 3, qui comprend au moins un peptide selon la revendication 1 et au moins un peptide selon la revendication 2.

5. Composition, selon la revendication 4, qui comprend un peptide ayant la SEQ ID n° 1 et un peptide ayant la SEQ ID n° 17.

6. Peptide, selon la revendication 1 ou 2, ou composition, selon l'une quelconque des revendications 3 à 5, destiné(e) à être utilisé(e) dans la suppression ou la prévention de la production d'anticorps inhibiteurs du facteur VIII *in vivo.*

7. Peptide, selon la revendication 1 ou 2, ou composition, selon l'une quelconque des revendications 3 à 5, destiné(e) à être utilisé(e) dans un procédé de traitement de l'hémophilie chez un sujet.

8. Peptide ou composition pour une utilisation selon la revendication 7, dans laquelle le sujet est atteint d'hémophilie A et subit ou va subir une thérapie de remplacement du facteur VIII et/ou une thérapie par dérivation du FVIII.

9. Peptide ou composition pour une utilisation selon la revendication 7, dans laquelle le sujet est atteint d'hémophilie acquise ou a un risque de la contracter.

10. Peptide ou composition pour une utilisation selon l'une quelconque des revendications 7 à 9, dans laquelle le sujet est HLA-DR2.
